Europäisches Patentamt

**European Patent Office**

**Office européen des brevets**

(19)

(11) Publication number: **0 240 224**

**A2**

(12) **EUROPEAN PATENT APPLICATION**

(21) Application number: **87302519.1**

(22) Date of filing: **24.03.87**

(51) Int. Cl.³: **C 12 N 15/00**
C 12 P 21/02, C 12 N 1/16
//(C12N1/16, C12R1:865)

(30) Priority: **31.03.86 US 845937**

(43) Date of publication of application:
**07.10.87 Bulletin 87/41**

(84) Designated Contracting States:
**AT BE CH DE ES FR GB GR IT LI LU NL SE**

(71) Applicant: **INTERFERON SCIENCES, INC.**
**783 Jersey Avenue**
**New Brunswick New Jersey 08901(US)**

(72) Inventor: **O'Loughlin, John T**
**158 West Acton Road**
**Stow, Massachusetts 01775(US)**

(74) Representative: **Bannerman, David Gardner et al,**
**Withers & Rogers 4 Dyer's Buildings Holborn**
**London, EC1N 2JT(GB)**

(54) An alpha interferon analogue.

(57) A novel alpha interferon analogue, lacking a diami-
nopeptidase site at positions 22–23, may be expressed intact
in hosts whose primary proteolytic activity against the
natural species is at that site. A preferred host is a
*Saccharomyces cerevisiae* strain carrying the PEP 3–4 muta-
tion. The analogue has a Ser, Thr, Asn, Gln or Gly at position
22.

EP 0 240 224 A2

-1-

## AN ALPHA INTERFERON ANALOGUE

### Field of Invention

This invention relates to an alpha interferon analogue which is resistant to a protease specific for ARG-ARG or ARG-LYS dibasic amino acid site at position 22 and 23 in many natural alpha interferon species.

### BACKGROUND OF THE INVENTION

In this application, standard interferon nomenclature is followed. Thus, the alpha interferons are a family of proteins having interferon activity which can be distinguished both immunologically and structurally from other interferons. Sequences for many alpha interferons are given by Pestka, Sci. Am. 249(2):37-43 (1983). For interferon nomenclature, see Nature, 286:110 (1980) and ASM News, 50:68 (1984).

Human interferon alpha-2 (alpha A) has a known primary sequence of 165 amino acids, typically with disulfide bridges coupling CYS 1 to CYS 98 and CYS 29 and CYS 138. Three allelic variants of alpha-2 have been sequenced:

(1) The LYS 23, HIS 34 variant of Goeddel, et al., Nature, 287:411-416 (1980), from KG-1 myeloblastoid cells;

(2) The ARG 23, HIS 34 variant of Streuli, et al., Science, 209:1343-47 (1980), from leukocytes; and

(3) The ARG 23, ARG 34 variant of Dworkin-Rastl, et al., Gene 21:237-48 (1983).

Interferons have been expressed in yeast, but without particular recognition of their susceptibility to diaminopeptidase activity in that cellular environment. Hitzeman, et al., Science, 219: 620 (1983; Kramer, et al., PNAS, 81:367 (1984); Dobson, et al., Nucl. Acids Res., 11:2287 (1987); Tuite, et al., EMBO J., 1:603 (1982); Lee, et al., Nucl. Acids Res., 12:6797 (1984); and Bowden, et al., Gene, 27:87 (1984).

There is significant interest in producing human alpha interferon in heterologous hosts, such as yeast. Because alpha interferon is not a natural yeast product, there is no guarantee that it will be resistant to attack by yeast proteolytic enzymes.

A number of proteolytic enzymes are found in yeast. Archstetter, et al., Arch. Biochem. Biophys., 207:445-54 (1981). The yeast mating pheromone alpha factor is expressed as a precursor protein which is processed by three enzymatic activities: trypsin-like or cathepsin B-like cleavage at Lys-Arg; carboxypeptidase B-like cleavage of basic residues from the excised peptides; and removal of the Glu-Ala or Asp-Ala dipeptides by dipeptidyl aminopeptidase A. The putative structural gene (KEX2) for the Lys-Arg cleaving endopeptidase was isolated by Julis, et al., Cell, 37:1075-89 (1984).

Bitter, et al., PNAS, 81:5330-34 (1984) constructed a fused gene comprising the yeast alpha-factor leader and a 166-amino acid alpha interferon analogue (IFN-alpha-Con). While internal cleavages did occur in the interferon moiety during alpha factor-directed secretion from yeast, over 95% of the interferon was secreted intact. The sites of cleavage were not identified.

Horwich, et al., PNAS, 82:4930-33 (1985) reported that the leader peptides of mitochondrial proteins were strikingly basic in overall amino acid composition. The simultaneous substitution of glycine for arginine at three positions in the leader peptide of the OTCase precursor resulted in the failure of the leader peptide to be cleaved by the matrix protease, even though the nearest arginine residue lay eight residues away from the site of proteolytic cleavage. Other studies have likewise suggested that amino acids remote from the cleavage site may affect proteolysis. Hurt, et al., FEBS Lett., 178:306-10 (1984); Horwich, et al., EMBO J., 4:1129-35 (1985). (Horwich speculates that the arginines contribute to a secondary or tertinary structure recognized by the matrix enzyme.) These observations illustrate the difficulty of predicting the susceptibility of a modified polypeptide sequence to proteolytic attack.

Similar concerns exist with respect to expression of interferon in other hosts. Braude, et al., Biochem. & Biophys. Comm., 89:612 (1979) reported that the proteolytic enzyme alpha-chymotrypsin, which predominantly cleaves the carboxyl side of tryptophyl phenylalanyl and tyrosyl residues, appeared to differentially inactivate interferon species.

It is known that alpha interferon is vulnerable to tryptic cleavage at ARG-ARG and ARG-LYS sites in vitro (US 4,503,035). While many tryptic cleavage sites were identified in vitro, it is unclear whether the interferon molecule is attacked at all of these sites in nature. We have found that the site at positions 22-23 is the primary cleavage site of a novel diaminopeptidase found in a protease-deficient yeast strain, and that cleavage at that site adversely affects biological activity.

Proteolysis would be expected to reduce the overall yield of active interferon. The proteolytic fragments complicate the purification process by increasing the aggregation tendencies of the interferon molecule (the active species is a monomer), and would be expected to induce, directly or indirectly, an immune response. Similar problems may be expected in other expression systems.

Until now, no one has recognized the importance of the ARG-LYS or ARG-ARG diaminopeptidase cleavage site at positions 22-23 in many alpha interferons or found a means of modifying these interferons to make them naturally resistant to such diaminopeptidase action without loss of biological activity.

Genetic modification is superior to the use of proteolytic enzyme inhibitors of protease deficient strains, or of subsequent purification steps to isolate intact, monomeric interferon.

Proteolytic enzyme inhibitors are known. (Pringle, in Methods in Cell Biology, Vol. 12.) It is undesirable to prevent proteolysis by adding proteolytic enzyme inhibitors, since these are highly toxic and must be removed if the interferon is to be used clinically. Additionally, these inhibitors are not always effective.

One may of course transform a host yeast system which is "protease-deficient." See Yeast Genetics: Fundamental and Applied Aspects 167-203 (1983) and Molecular Biology of the Yeast Saccharomyces 74-75 (1982). However, as we found in the case of one such strain, the elimination of the major proteolytic enzymes may simply unmask other, more specific proteolytic enzymes, such as the one newly characterized herein.

## SUMMARY OF THE INVENTION

We have found that a strain of Saccharomyces cerevisiae, carrying the pep 3-4 mutation, which results in deficiency of several major proteases, exhibits an ARG-ARG and ARG-LYS endoproteolytic activity that could not be detected in the presence of normal protease levels. This protease cleaves 19K interferon to generate a 16.25K fragment. This activity was observed when a recombinant interferon alpha, expressed in this strain, was found to undergo specific proteolysis. We identified the primary cleavage site as being the dibasic peptide ARG 22 - ARG 23. (Known species of alpha-2 interferon are either ARG 22 - ARG 23 or ARG 22 - LYS 23.)

During the production of the recombinant molecule by the host organism the presence of the dibasic site leads to a specific endoproteolytic event which removes the amino terminal 22 amino acids from the molecule. This event has been observed in yeast, E. coli, and mammalian systems. Thus, Bodo and Fogy, "Characterization of Different Molecular Species in Affinity Purified Recombinant Human Interferon Alpha 2", in The Interferon System, Rome, Italy, May 8-11, 1985, fractionated recombinant alpha-2 interferon (ARG 23, ARG 34) expressed in E. coli, into seven peaks, one of which, K7, was identified as alpha-2 IFN (23-165) in reduced form. Neither of the products of this proteolysis showed any antiviral activity as determined by standard interferon bioassays.

The affinity purified products from the yeast transformed with an alpha interferon expression vector may contain up to 14% (determined by coomasie blue staining) proteolytically processed interferon in the final product. The presence of this site on the interferon molecule directly leads to a significant reduction in the total yield of active interferon obtained from the yeast lysate.

Further, the presence of the truncated interferon in the product greatly complicates the purification process.

The aggregation tendencies of the interferon molecule are well documented. The primary cause of this phenomenon appears to be "scrambling" of the molecules normal intermolecular disulfide bonds leading to the formation of intramolecular disulfide bridges.

All interferons with exception of alpha-1 have 4 cys residues leading to the formation of 2 internal disulfide bridges. The presence of the large proteolytic fragment in the product mixture introduces a molecule with a free cys residue. This free cys residue may exacerbate the aggregation tendencies of the product. Correlative evidence for this is provided by the fact that when the product is analyzed under nonreducing and nondenaturing conditions the large proteolytic fragment is found exclusively in the aggregate population.

It has been demonstrated that the active species of the alpha IFN molecule is a monomer. Thus, since proteolysis enhances formation of the inactive aggregates, it indirectly leads to further reduction in the overall yield.

Additionally, the large proteolytic fragment may directly or through the induced aggregation lead to an increased antigenicity of the interferon product.

Therefore it is desirable from both specific activity and immunogenicity standpoints to purify the monomeric interferon away from the proteolytic fragments and the aggregate forms associated with them. This requires that a sophisticated and costly HPLC purification step be inserted into the normal purification scheme. Alternatively, oligomeric IFN may be reduced to monomeric form. (US 4,432,895 and EP Appl. 128,467).

An alternate approach is the prevention of fragment formation. Generally, proteolytic processing of recombinant products by host enzymes has been addressed through the use of protease inhibitors and/or by using host organisms deficient for the major proteases. In the production of recombinant pharmaceuticals the introduction of protease inhibitors which are (highly toxic) presents a serious difficulty. The often difficult removal of the material to acceptable levels must be demonstrated. Thus, it does not represent a preferred approach. The use of proteolytically deficient strains normally represents an effective strategy. In the case of interferon however, the structure of the molecule itself seems to play a more significant role. This normally effective method is not sufficient to fully protect the product molecule. This is substantiated by the degree of interferon proteolysis that we observe in our own host organism; a strain of Sachacromyces wherein 70% of the activity of the major proteases is absent.

Clearly, a technique which would prevent the formation of these proteolytic fragments would be a great benefit during both the harvesting and purification stages of interferon production.

Since the primary cleavage site of the aforementioned protease in our preferred host is at the ARG-ARG or ARG-LYS site at positions 22-23, we attempted to identify modifications which would be unlikely to adversely affect biological activity. As will be explained in greater detail hereafter, a substitution of Ser, Thr, Asn, Gln, or Gly at position 22 would destroy the dibasic site while still preserving the conformation of the active molecule.

Of the characterized alpha interferons, Alpha-2 is ARG-LYS at positions 22-23; Alpha-6, Alpha-8 and Alpha-H are ARG-ARG; Alpha-1 is SER-ARG; Alpha-C, Alpha-F, Alpha-5, Alpha-I, Alpha-7, and Alpha-10 are GLY-ARG; and Alpha-4B is GLY-LYS. Pestka, Sci. Am., 249(2): 37, 42 (August 1983).

While the alpha interferon analogue of this invention is preferably expressed in a host system where the primary ARG-ARG or ARG-LYS cleavage activity against the natural species is at positions 22-23, it may be of value in other systems by virtue of reducing the number and variety of proteolytic cleavage products which must be excluded by subsequent purification procedures. The 16K cleavage product of unique cleavage at the position 22 site of alpha IFN has been particularly implicated in the formation of undesirable aggregates.

BRIEF DESCRIPTION OF THE DRAWINGS

Fig. 1 shows an analysis of the amino terminals of the 19K interferon and its 16.25K fragment.

Fig. 2 shows the known amino acid sequence of the recombinant alpha-2 interferon, with the cleavage site marked.

## DETAILED DESCRIPTION OF THE INVENTION

Yeast lysates were prepared from strains of S. cerevisiae containing the PEP 3-4 Protease mutations (ISI013) and the URA 3-50, GAL, and GAL 80 mutations (CGY 306). Cells were lysed with a bead beater at 4'C in 10 mM EDTA, 100 mM HEPES pH 7.5, and optionally 0.5% TRITON X-100. The lysate was clarified by centrifugation at 13,000 x g for 2 minutes, aliquoted into 40 ul portions and stored frozen at -70'C.

Each lysate was prepared in the presence or absence of 0.5% TRITON X-100 (+,-) and an aliquot of each was clarified by centrifugation.

The lysates were "spiked" with recombinant alpha -2 IFN prepared as described which is less than 4% in the "16.25K" peptide fragment. IFN levels in the lysate were adjusted to reflect concentrations routinely obtained in actual batch lots, i.e. approx. $2 \times 10^6$ U/ml. The lysates were incubated for 16 hours at 25'C. A portion of the lysates was removed and analysed by Western blotting with an anti-alpha-2 IFN antibody.

It is apparent that the presence of TRITON X-100 corresponds with that of the 16.25K fragment. Clarification of the lysate by centrifugation appears to have no effect. The fragment cannot be detected in the CGY306 strain which contains normal levels of Protease.

We have partially purified the enzyme responsible for the proteolysis of alpha-2 interferon in our yeast strains. It is membrane bound and activated by the presence of TRITON X-100 during lysis of the cells. The activity is not effected by a broad range of protease inhibitors that we examined. Its characteristics lead us to believe that it is a novel enzyme yet to be described in the literature.

The degree of purity we have obtained allowed us to explore the substrate specificity within the alpha interferon family. We have isolated the products and performed detailed analysis of the cleavage site.

Recombinant alpha-2, alpha-1, and alpha-8 IFN were produced in yeast and purified by affinity chromotography on specific monoclonal antibody columns, followed by size exclusion HPLC.

Protease digestions of specific alpha IFNs were performed as follows: 1 ug of alpha IFN was added to 40 ul of yeast lysate, both prepared as indicated. The mixture was incubated at 25'C for 16 hours. (Cleavage is first seen at about one hour.) Following incubation, 6 ul of sample was added to 20 ul of Laemmli sample buffer. The samples were heated at 100'C for 5 minutes and electrophoresed on a 20% acrylamide gel under reducing conditions.

The gel was electroblotted to nitrocellulose and the pattern developed by the immuno-staining method of DeBlos, and Cherwinski, Anal. Biochem. 133: 214-219 (1983). The primary antibodies employed in the staining procedure were those used in the affinity chromotography of the alpha IFN in question.

A series of experiments were performed using alpha-2 interferon as substrate for the enzyme. Recombinant alpha-2 IFN (prepared as described) was subjected to reverse phase HPLC on a C18 U Bordopok column (WATERS) and developed with a linear gradient of acetonitrile from 0 to 95% in one hour in the presence of .1% TFA.

Fractions 1-3 were collected and analysed by SDS acrylamide gel electrophoresis (according to Laemmli) under both reducing (C) and nonreducing (B) conditions.

The major peak (Fraction 1) appears as an approximate 19K monomer under both reducing and nonreducing conditions. Fraction 2, analysed under nonreducing conditions appears as a mixture of monomer and dimer. Under reducing conditions the presence of the 16.25K fragment in Fraction 2 is evident.

Peak 3 analysed under nonreducing conditions shows the presence of monomer, dimer and higher order aggregates. Reducing conditions indicate that the 16.25K fragment is also present in this population.

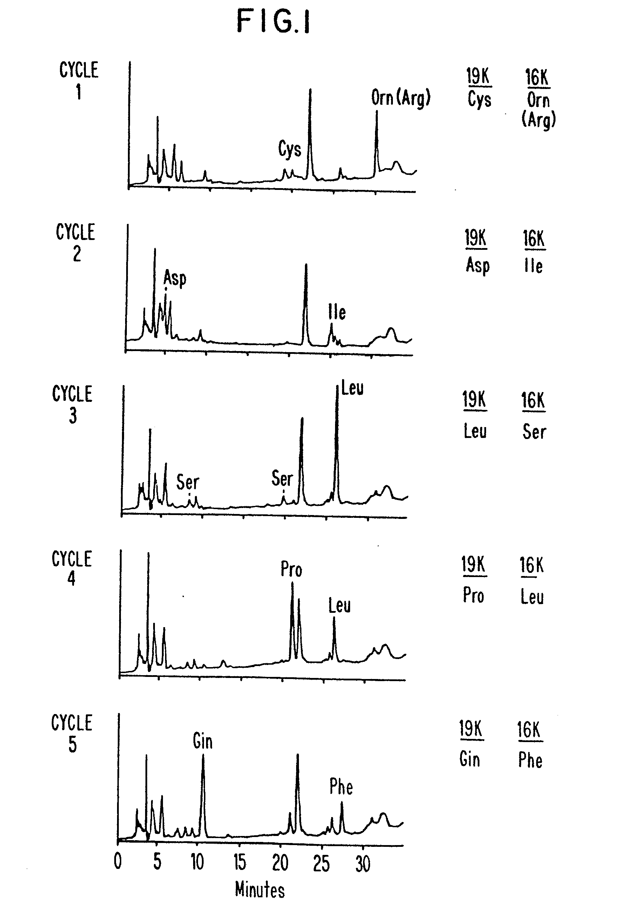

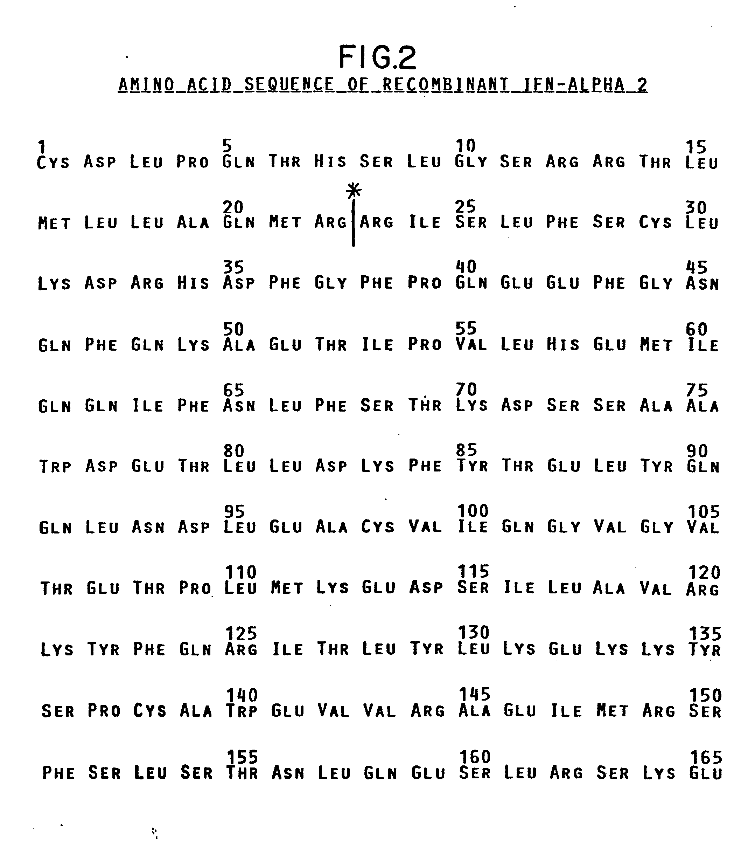

The reaction products were analyzed both chemically and enzymatically to determine their amino acid sequence. We found that the interferon molecules are hydrolysed at the peptide bond between positions 22 and 23, both occupied by ARG (Figs. 1 and 2). Further, during the cleavage process, ARG 23 is modified, resulting in an ORN residue in the nascent amino terminal position.

The experiment was extended with the use of alpha-8 interferon as substrate for the enzyme. It is the only other member of the alpha interferon family known to have the ARG-ARG configuration at positions 22-23. As expected, this molecule was also hydrolysed in a manner similar to that observed with alpha-2 interferon.

Basic pairs of amino acids represent typical trypsin-like cleavage sites. Specificity for a particular cleavage site is a characteristic of many proteolytic enzymes. We therefore concluded that modification of the 22nd or 23rd position might greatly lower the affinity of the enzyme for this molecule.

This hypothesis was analyzed by using alpha-1 interferon as substrate for the enzyme.

Alpha-1 interferon, among other differences, contains a SER-ARG at positions 22-23. After incubation of this substrate with the protease no hydrolysis whatever could be detected. Seemingly then, the substitution of SER for ARG at position 22 inhibits the proteolytic degradation of the alpha interferon molecule at that site by the novel yeast diaminopeptidase noted above.

In summary then, we have data which strongly suggests that a modification of alpha-2 interferon at position 22 will render it resistant to proteolysis at that position. Further, there is no biochemical reason to restrict the substitution to only SER as found in alpha-1 interferon.

The codon used for ARG 22 in the alpha-2 interferon gene is AGG. The substitution of single base changes within this codon (statistically the most likely event in nature) leads to the possibility of seven different residues at position 22 (Arg, Gly, Lys, Ser, Met, Trp, Thr). Only four of these possibilities actually occupy position 22 in the native molecule. The residues observed in nature are Lys, Arg, Gly, and Ser. These substitutions involve both purine to purine and purine to pyrimidine base changes. However, all the resulting amino acids are characterized by polar R groups. Therefore, there may be some selective pressure for maintaining a polar amino acid at position 22.

Two of the polar residues (Arg and Lys) must be ruled out for use in this modification because of the positive charge on their R group. Their use would simply recreate the diaminopeptidase recognition site. Two more (Glu and Asp) are rejected because of their negatively charged side group. There is no known alpha interferon with a negatively charged amino acid in this position, and such residues may interact with other residues (e.g., by salt bridges) in an undesirable manner. Ser is found at position 22 in only one of the known alpha interferons. Many alpha interferon species have Gly in this position.

With the only apparent constraint being a polar uncharged R group, six amino acids become candidates for the substitution (Ser, Thr, Cys, Tyr, Asn and Gln). Consideration of tertiary structure implications leads to the disapproval of Cys and Tyr. Of the remaining residues, the presence of Ser in the native alpha interferons favors its selection. However, there is no reason to assume that Thr, Asn or Gln would be any less suitable for the purpose. To this list one may also add the nonpolar amino acid glycine, in view of its presence in several alpha interferons in this position.

The five preferred possibilities (Ser, Thr, Asn, Gln, Gly) can all be easily tested by site specific mutagenesis of alpha-2 interferon using the M13 bacteriophage system. The DNA sequence of our alpha-2 interferon clone has been determined. This greatly simplifies the choice of the required oligonucleotide primer sequences. The remainder of the M13 mutagenesis technique involves standard procedures.

There are many vectors available that are derived from the single stranded phage M13. They have been applied to oligonucleotide directed site specific mutagenesis and provide simple, rapid isolation of single stranded DNA.

Several strains of M13 developed by Messing (Meth. Enzymol. 101[2] (1984)) are commercially available and the basic strategy for cloning a fragment into M13 is described by him.

For mutagenesis of alpha-2 IFN, a HindIII fragment containing the entire coding region can be inserted into the HindIII site of several commercially available M13 vectors e.g. MP 8, 9, 10, 11, 18, 19. For a detailed discussion of M13 cloning see Wasylyk, et al., PNAS 77:7024 (1980).

The orientation and strand of the insert can be determined by Di-Deoxy sequencing using a commercially available M13 universal primer. A clone of the desired strand can be chosen and used as a source of single stranded template. For example the (-) strands of the alpha IFN insert may be used.

Examples of some oligonucleotides designed to direct the described mutagenesis and their hybridization to the (-) strand of alpha-2 IFN in the area of interest are listed below.

|  |  | ALA | GLN | MET | ARG* | ARG | ILE | SER |  |
|---|---|---|---|---|---|---|---|---|---|
| alpha-2 IFN (-) | 5' | GCA | CAG | ATG | AGG | AGA | ATC | TCT | 3' |
| FOR ARG* to: |  |  |  |  |  |  |  |  |  |
| Gly | 3' | CGT | GTC | TAC | CCA | TCT | TAG | AGA | 5' |
| Ser | 3' | " | " | " | TCA | " | " | " | 5' |
| Thr | 3' | " | " | " | TGC | " | " | " | 5' |
| Asn | 3' | " | " | " | TTG | " | " | " | 5' |
| Gln | 3' | " | " | " | GTT | " | " | " | 5' |

The desired mutagenic olegonucleotide and the single stranded template DNA are annealed and extended by the large fragment of DNA polymerase. This incubation is generally carried out for a period of 12-20 hours at 15'C. After the primer is extended around the circular template the two ends of the newly synthesized strand are ligated by T4 DNA ligase.

The resulting closed circle heteroduplex DNA is then used to transform competent JM 101 cells as described by Sanger, et al., J. Mol. Biol. 143:161 (1981).

The single stranded phage are isolated and examined by an appropriate screening procedure, in the case of alpha-2 IFN sequencing by the Di-Deoxy method.

It is not intended, however, that applicants be limited to any particular method of providing an alpha interferon analogue, which lacks an ARG-ARG or ARG-LYS proteolytic cleavage site at positions 22-23.

While we prefer to modify position 22 in order to destroy the diaminopeptidase recognition site, we would not rule out the possibility of modifying position 23 while still preserving biological activity. The sequence data for the alpha interferon family, however, provides less guidance as to allowable modifications of position 23.

The analogue herein described is preferably expressed in a host whose primary proteolytic activity against alpha interferons is at the ARG-ARG or ARG-LYS site at positions 22-23. This may be a _S. cerevisiae_ carrying the PEP3-4 mutation, or it may be in another host which is deficient in other proteases.

CLAIMS

1. An alpha interferon analogue having alpha interferon activity, and lacking a dibasic diaminopeptidase recognition site at positions 22 and 23, said site being found in the analogous natural interferon species.

2. The analogue of claim 1 in which the site is ARG-ARG or ARG-LYS.

3. The analogue of claim 1, having a polar uncharged amino acid selected from the group consisting of Serine, Threonine, Asparagine and Glutamine, at position 22.

4. The analogue of claim 3, having a Serine at position 22.

5. The analogue of claim 2, having a Glycine at position 22.

6. The analogue of claim 2 in which the analogue is of interferon alpha -2.

7. The analogue of claim 2 in which the analogue is of interferon alpha -8.

8. The analogue of claim 1, said analogue being expressible in intact form in a Saccharomyces cerevisiae carrying the PEP 3-4 mutation.

9. A method of producing a protein having alpha interferon activity which comprises transforming a host with a vector carrying genetic information encoding an analogue according to claim 1 under conditions favorable to expression of the information, and collecting the expressed analogue.

10. The method of claim 9 in which the host cell's primary proteolytic activity against the corresponding natural alpha interferon is at positions 22-23.

11. The method of claim 10 in which the host is a Saccharomyces cerevisiae carrying the PEP 3-4 mutation.

12. An expression vector capable of expressing the analogue of claim 1 in a suitable host.

13. A host transformed by the expression vector of claim 12.

# FIG.I

CYCLE 1 — 19K Cys — 16K Orn (Arg)

CYCLE 2 — 19K Asp — 16K Ile

CYCLE 3 — 19K Leu — 16K Ser

CYCLE 4 — 19K Pro — 16K Leu

CYCLE 5 — 19K Gln — 16K Phe

Minutes

# FIG.2
## AMINO ACID SEQUENCE OF RECOMBINANT IFN-ALPHA 2

CYS ASP LEU PRO GLN THR HIS SER LEU GLY SER ARG ARG THR LEU

MET LEU LEU ALA GLN MET ARG|ARG ILE SER LEU PHE SER CYS LEU

LYS ASP ARG HIS ASP PHE GLY PHE PRO GLN GLU GLU PHE GLY ASN

GLN PHE GLN LYS ALA GLU THR ILE PRO VAL LEU HIS GLU MET ILE

GLN GLN ILE PHE ASN LEU PHE SER THR LYS ASP SER SER ALA ALA

TRP ASP GLU THR LEU LEU ASP LYS PHE TYR THR GLU LEU TYR GLN

GLN LEU ASN ASP LEU GLU ALA CYS VAL ILE GLN GLY VAL GLY VAL

THR GLU THR PRO LEU MET LYS GLU ASP SER ILE LEU ALA VAL ARG

LYS TYR PHE GLN ARG ILE THR LEU TYR LEU LYS GLU LYS LYS TYR

SER PRO CYS ALA TRP GLU VAL VAL ARG ALA GLU ILE MET ARG SER

PHE SER LEU SER THR ASN LEU GLN GLU SER LEU ARG SER LYS GLU